# EUROPEAN PATENT APPLICATION

(11) **EP 1 044 979 A2**
(43) Date of publication of application: **18.10.2000**
(21) Application number: 00400795.1
(22) Date of filing: 23.03.2000
(51) Int. Cl.: C07D 498/10, C07D 209/04, G02B 5/23, C09K 9/02

(54) **1-[(N-(un)substituted)amidoalkyl]spiroindolinonaphthoxazines, their preparation, compositions and (co)polymer matrices containing them**

(30) Priority: 24.03.1999 RU 99106201
(71) Applicant: Corning S.A., 77920 Samois sur Seine (FR)
(72) Inventor: Gromov, Sergei Panteleimonovich, Moscow 119634 (RU); Sergeev, Sergei Anatoljevich, Moscow 115583 (RU); Fedorova, Olga Anatoljevna, Moscow 119634 (RU); Strokach, Yury Petrovich, Moscow Region 141700 (RU); Dmitrieva, Svetlana Nikolaevna, Moscow 131165 (RU); Barachevsky, Valery Alexandrovich, Moscow 111672 (RU); Alfimov, Mikhail Vladimirovich, Moscow 109072 (RU)
(74) Representative: Le Roux, Martine

(57) **Abstract**

The object of the present invention is novel spiroindolinonaphthoxazine compounds as well as the compositions and (co)polymer matrices containing them. Said compounds have interesting photochromic properties. Another object of the present invention is a method of preparing said novel compounds.

## Description

The present invention relates to novel spiroindolinonaphthoxazine compounds, more particularly 1-[(N-(un)substituted)amidoalkyl]-spiroindolinonaphthoxazines, which have, in particular, photochromic properties. The invention also relates to:
- the preparation of such compounds ;
- photochromic compositions and photochromic ophthalmic articles (lenses for example) which contain said spiroindolinonaphthoxazines
- intermediate compounds useful in their preparation.

The photochromic compounds are capable of changing colour under the influence of a poly- or monochromatic light (UV for example) and of returning to their initial colour when the luminous irradiation ceases, or under the influence of temperature and/or a poly- or monochromatic light different from the first.

The photochromic compounds find applications in various fields, e. g. for the manufacture of ophthalmic lenses, contact lenses, solar protection glasses, filters, camera optics or photographic apparatus optics or other optical devices and observation devices, glazing, decorative objects, bill elements or even for information storage by optical inscription (coding).

In the field of ophthalmic optics, and in particular the spectacles trade, a photochromic lens which comprises one or more photochromic compounds must have:
- a high transmission in the absence of ultraviolets,
- a low transmission (high colorability) under solar irradiation,
- adapted coloration and discoloration kinetics,
- a tint acceptable to the consumer (grey or brown preferably) with preferably a maintenance of the chosen tint during the coloration and the discoloration of the lens,
- a maintenance of the performances, the properties, within a temperature range of 0-40°C,
- a significant durability, since these objectives sought after are sophisticated corrective lenses and therefore expensive.

These lens characteristics are in fact determined by the active photochromic compounds which they contain; compounds which must furthermore be perfectly compatible with the organic or inorganic support which constitutes the lens.

Moreover, it is to be noted that obtaining a grey or brown tint may necessitate the use of at least two photochromes of different colours, i. e. having distinct maximal absorption wavelengths in the visible. This association further imposes other requirements of the photochromic compounds. In particular, the coloration and discoloration kinetics of the (two or more) associated active photochromic compounds must be essentially identical. The same applies for their stability with time and also for their compatibility with a plastic or inorganic support.

Amongst the numerous prior art documents describing spironaphthoxazines, the following may be cited in particular :
- EP-A-0 313 941 (Nissan Motor Co., Ltd) discloses a photochromic material to be used for a photosensitive arrangement such as photosensitive laminated glass. The photochromic material is formed of a high polymer substrate material containing photochromic (hetero)aromatic spiroxazine compounds bearing a (hetero)indole moiety which is substituted on the N-atom with an alkyl, alkenyl, cycloalkyl or aryl group.
- FR-A-2,738,570 (Corning Incorporated) discloses photochromic spiroxazines. These compounds are similar in structure to those disclosed herein but differ substantially about the substituent borne on the N-atom of the indoline ring which is a dissymmetric aliphatic monocyclic group.
- FR-A-2,738,248 (Corning Incorporated) also discloses photochromic spiroxazines, only this time the substituent borne by the N-atom of the indoline ring is a polycyclic group formed by at least one alicyclic group linked, bridged or condensed with at least one other aliphatic and/or aromatic ring *inter alia.*
- EP-A-0 600 669 (Pilkington PLC) discloses photo-reactive materials of spiro(hetero)indolinonaphthoxazine structure on the N-atom of the indoline ring of which is a (cyclo)alkyl- or (hetero)aryl-substituted methylene group.
- US-A-4,756,973 (Kureha Kagaku Kogyo Kabshiki Kaisha) discloses a photochromic lens which comprises a spiroindolinonaphthoxazine structure on the N-atom of the indoline ring of which is an alkyl group which may itself be substituted with a carboxylic acid group, a cyano group, a substituted arylalkyl group, or an ester group.
- Finally, EP-A-0 171 909 (Toray Industries, Inc.) discloses similar spiroindolinonaphthoxazine structures on the N-atom of the indoline ring of which is an alkyl group which may itself be substituted with a phenyl or naphthyl group.

It is to the credit of the Applicant to have discovered a novel type of spiroindolinonaphthoxazine compound which possesses particularly advantageous photochromic properties. It is also to the credit of the Applicant to have proposed a means of access (an efficient synthesis method) of preparing such novel spiroindolinonaphthoxazine compounds. More specifically, said compounds possess high λₘₐₓ and a high colorability, associated with rapid discoloration kinetics.

Thus, the object of the present invention is novel compounds of formula (I): 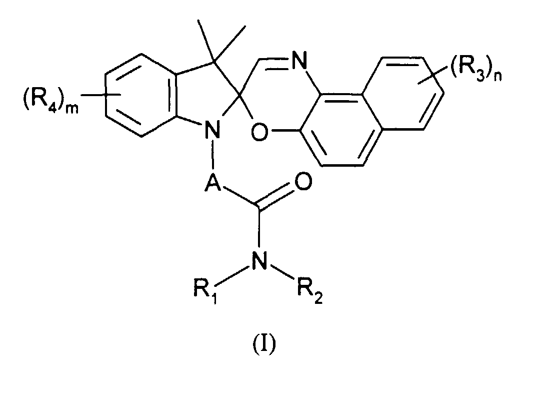 in which :
- A represents :
   - a linear alkyl group having 3 to 12 carbon atoms, advantageously from 3 to 7 carbon atoms, or
   - a branched alkyl group-containing alkyl group having 4 to 12 carbon atoms of formula:

      ―(CH₂)ᵣ―X―(CH₂)ₛ―

      in which r is equal to or greater than 1, s is equal to or greater than 1, and X represents said branched alkyl group ;
- R₁ and R₂, identical or different, independently represent :
   - hydrogen,
   - a linear alkyl group having 1 to 4 carbon atoms, advantageously 1 or 2 carbon atoms,
   - a cycloalkyl group having 3 to 12 carbon atoms,
   - an aryl or aryloxy group having 6 to 24 carbon atoms in its basic structure, or a heteroaryl or heteroaryloxy group having 4 to 24 carbon atoms in its basic structure and at least one heteroatom selected from sulphur, oxygen and nitrogen ; said basic structures optionally being substituted with at least one substituent selected from :
      + a halogen and notably fluorine, chlorine and bromine,
      + a linear or branched alkyl group having 1 to 6 carbon atoms,
      + a linear or branched alkoxy group having 1 to 6 carbon atoms,
      + a linear or branched haloalkyl or haloalkoxy group having 1 to 6 carbon atoms, and notably a fluoroalkyl group of this type, advantageously a trifluoromethyl group,
      + a linear or branched alkenyl or alkynyl group having 2 to 12 carbon atoms and notably a vinyl group or an allyl group,
      + an -NO₂ group,
      + an -NH₂ group,
      + an -NHR group, R representing a linear or branched alkyl group having 1 to 6 carbon atoms.
      + a 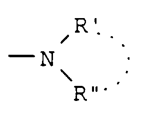 group, R' and R", identical or different, representing independently a linear or branched alkyl group having 1 to 6 carbon atoms or representing, together with the nitrogen atom to which they are bound, a 5- to 7-membered ring which can comprise at least one other heteroatom selected from oxygen, sulphur and nitrogen, said 5- to 7-membered ring being non-substituted or substituted with an R"' group, said R"' group being selected from a linear or branched alkyl group having 1 to 6 carbon atoms, an amine group, and a nitro group ; in the case in which said other heteroatom is nitrogen, said nitrogen may optionally be substituted with a linear or branched alkyl group having 1 to 6 carbon atoms,
      + a methacryloyl group or an acryloyl group,
      + an epoxy group of formula : 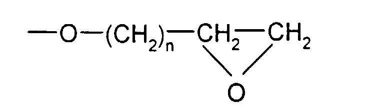 in which n = 1, 2 or 3,
   - an aralkyl, aryloxyalkyl, heteroaralkyl or heteroaryloxyalkyl group, the linear or branched alkyl group having 1 to 4 carbon atoms and the aryl, aryolxy, heteroaryl, and heteroaryloxy groups having the definitions given above,
      or
   - said two substituents R₁ and R₂ together form with the N-atom to which they are bound, a 5- to 7-membered ring which can comprise at least one other heteroatom selected from oxygen, sulphur and nitrogen, said 5- to 7-membered ring being non-substituted or substituted with an R"' group, said R"' group being selected from a linear or branched alkyl group having 1 to 6 carbon atoms, an amine group, and a nitro group ; in the case in which said other heteroatom is nitrogen, said nitrogen may optionally be substituted with a linear or branched alkyl group having 1 to 6 carbon atoms, said two substituents R₁ and R₂ , together with the N-atom to which they are bound, representing advantageously a morpholino, piperidino, piperazino, or an indolino group ;
- R₃ and R₄ identical or different, independently represent:
   - a halogen, and notably fluorine, chlorine or bromine,
   - a linear or branched alkyl group having 1 to 12 carbon atoms (advantageously 1 to 6 carbon atoms),
   - a cycloalkyl group having 3 to 12 carbon atoms,
   - a linear or branched alkenyl or alkynyl group having 2 to 12 carbon atoms,
   - a linear or branched alkoxy or alkylthio group, having 1 to 12 carbon atoms (advantageously 1 to 6 carbon atoms),
   - a haloalkyl, a halocycloalkyl or haloalkoxy group corresponding respectively to the alkyl, cycloalkyl, alkoxy groups above, substituted with at least one halogen atom, notably selected from fluorine, chlorine and bromine,
   - an electron-withdrawing group, such as a CN, NO₂ or SCN group,
   - an aryl, aryloxy, heteroaryl or heteroaryloxy group having the same definition as that given above for R₁, R₂,
   - an aralkyl, aryloxyalkyl, heteroaralkyl or heteroaryloxyalkyl group, the linear or branched alkyl group having 1 to 4 carbon atoms and the aryl, aryloxy, heteroaryl, and heteroaryloxy groups having the same definitions as those given above for R₁, R₂,
   - an amino or amido group: -NH₂, - NHR, - CONH₂, - CONHR, 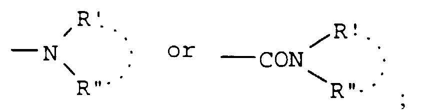 R, R', R" having their respective definitions given above for the amine substituents of the R₁, R₂ values: aryl or heteroaryl,
   - an - OCOR₆ or -COOR₆ group, R₆ representing a hydrogen atom, or a straight or branched alkyl group having 1 to 6 carbon atoms or a cycloalkyl group having 3 to 6 carbon atoms or a phenyl group optionally substituted with at least one of the substituents listed above for the R₁, R₂ values: aryl, aryloxy, heteroaryl or heteroaryloxy ; or
   - in the case in which m is equal to or greater than 2 in (R₄)ₘ , and/or in the case in which n is equal to or greater than 2 in (R₃)ₙ, said radicals, being preferably borne by two adjacent carbon atoms, can combine to form at least one aromatic ring having 5 or 6 members, or an aliphatic ring having 5 to 7 members, preferably 5 or 6 members, said ring(s) comprising, optionally, at least one heteroatom selected from sulphur, oxygen and nitrogen, so as to form at least one heterocyclic ring, the latter being optionally substituted by one or more radicals, which may be identical or different, and have the same definition as given above for R₄ and R₃ ;
- m is an integer of 0 to 4 ; and
- n is an integer of 0 to 6.

As regards R₃ and R₄, it must be understood that they represent, respectively, optional substituent(s) on the naphthyl ring, wherein there are six possible substitutable positions, or on the phenyl ring of the indolino moiety, wherein there are four possible substitutable positions. Said substituents may exist independently (i.e. at least one of m and n ≠ 0) or do not exist at all (i.e . at least one of n and m = 0). When at least two substituents R₃ and R₄ does exist, they may be identical or different.

Preferred compounds of the present invention are those of formula (I') below in which m = n = 0, and A, R₁ and R₂ are as defined for formula (I).

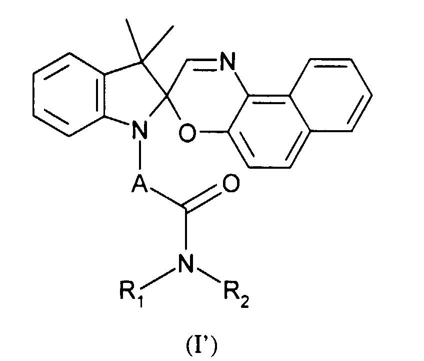

Particularly preferred compounds of the present invention are those of formula (I') above in which R₁ or R₂ is a substituted or unsubstituted aryl group, advantageously a substituted or unsubstituted phenyl group.

The compounds of formula (I) or (I') which are more preferred still are selected from the group consisting of the following :
3,3-dimethyl-1-[(N-phenyl)aminocarbonyltrimethylene]-spiro-[indolino-2,3'-[3H]-naphtho-[2,1-b]-[1,4]oxazine] : 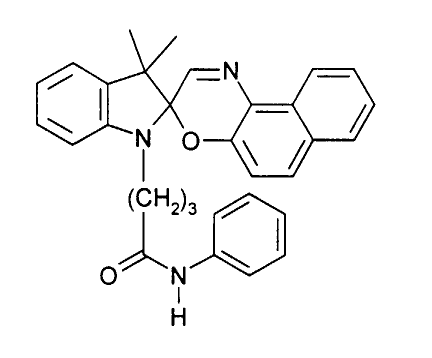
3,3-dimethyl-1-[(N-methyl-N-phenyl)aminocarbonylpentamethylene]-spiro-[indolino-2,3'-[3H]naphtho-[2,1-b]-[1,4]oxazine] : 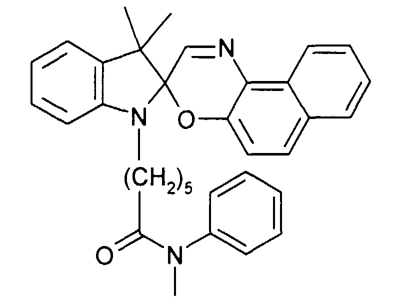
3,3-dimethyl-1-{[N-(4-trifluoromethylphenyl)]-aminocarbonylpentamethylene}-spiro-[indolino-2,3'-[3H]naphtho[2,1-b]-[1,4]oxazine] : 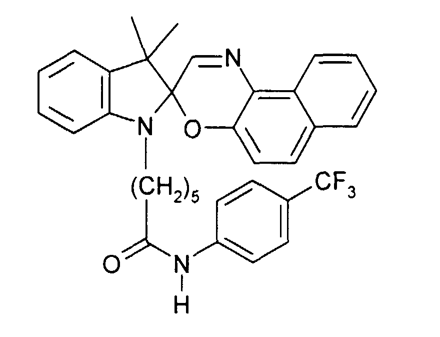
3,3-dimethyl-1-{[N-(4-methoxyphenyl)]aminocarbonylpentamethylene}-spiro-[indolino-2,3'-[3H]naphtho-[2,1-b]-[1,4]oxazine] : 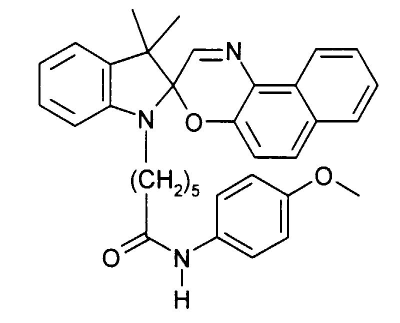
3,3-dimethyl-1-{[N-(4-nitrophenyl)]aminocarbonylpentamethylene}-spiro-[indolino-2,3'-[3H]naphtho-[2,1-b]-[1,4]oxazine] : 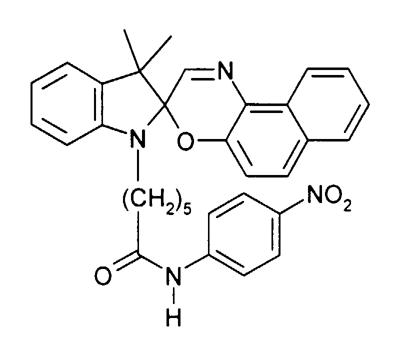
3,3-dimethyl-6'-morpholino-1-[(N-phenyl)aminocarbonylpentamethylene]-spiro-[indolino-2,3'-[3H]naphtho-[2,1-b]-[1,4]oxazine] : 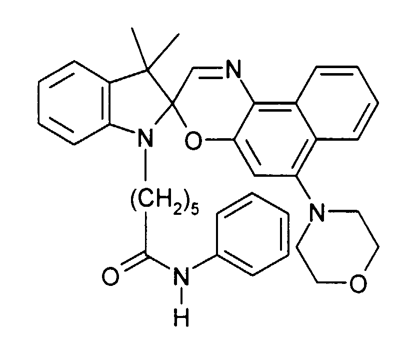
1-(aminocarbonylpentamethylene)-3,3-dimethyl-spiro-[indolino-2,3'-[3H]naphtho-[2,1-b]-[1,4]oxazine] : 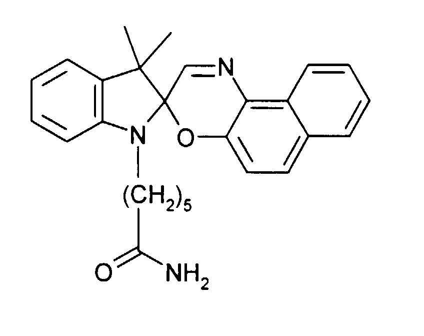
3,3-dimethyl-1-[(N-phenyl)aminocarbonylheptamethylene]-spiro-[indolino-2,3'-[3H]naphtho-[2,1-b]-[1,4]oxazine] : 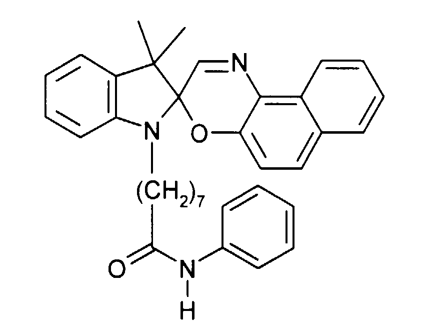
3,3-dimethyl-1-[(N-phenyl)aminocarbonyltetramethylene]-spiro-[indolino-2,3'-[3H]naphtho-[2,1-b]-[1,4]oxazine] : 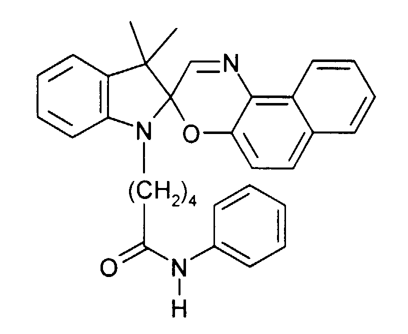
3,3-dimethyl-1-[(N-phenyl)aminocarbonylpentamethylene]-spiro-[indolino-2,3'-[3H]naphtho-[2,1-b]-[1,4]oxazine] :

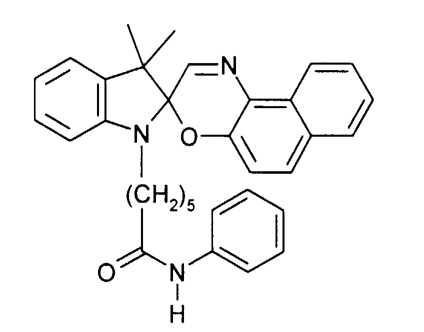

Amongst the substituents (R₁, R₂, R₃ and R₄) of the compounds of the invention, they are some which comprise and/or form at least one polymerizable and/or cross-linkable reactive group. The presence of such reactive groups can prove to be advantageous. Thus, the present invention includes, in its first object, spironaphthoxazine compounds, such as defined above, whose structure includes at least one polymerisation and/or cross-linking reactive group; said group consisting of an alkenyl or alkynyl group, advantageously vinyl or allyl, or of a methacryloyl, acryloyl or epoxy group.

Thus, the compounds of the invention which belong to this class can be grasped as monomers, of different nature or not, which can react with themselves and/or with other co-monomers in order to form homopolymers and/or copolymers which are carriers of a photochromic functionality (insofar as said monomers of the invention bear said photochromic functionality) and possess the mechanical properties of macromolecules.

It follows that another object of the present invention is formed by these linear or branched homopolymers or copolymers, at least in part constituted by the compounds of the invention.

Similarly, the above-mentioned compounds of the invention can be envisaged as cross-linking agents having reactive functions which can allow bridges between chains of photochromic or non-photochromic polymers. The reticulates (products of cross-linking) which can be obtained also constitute another object of the present invention.

The compounds of the invention - 1-[(N-(un)substituted)amidoalkyl]-spiroindolinonaphthoxazines of formula (I) - can be obtained in a general manner by :
a) allowing an indolenine derivative of formula (II) : 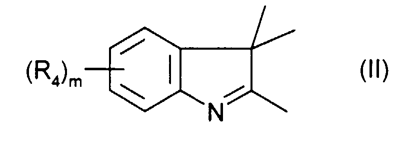 in which R₄ and m are as defined above, to react, with heating, with an electrophilic compound of formula (III): 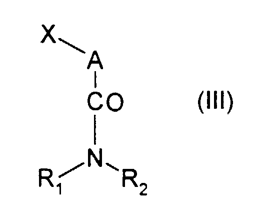 in which A, R₁ and R₂ are as defined above, and X is a leaving group, such as a halogen, for example, to provide an amide of formula (IV) : 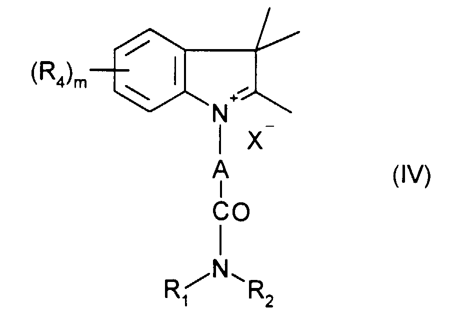
b) allowing said amide of formula (IV) to react with a 1-nitroso-2-naphthol derivative of formula (V) : 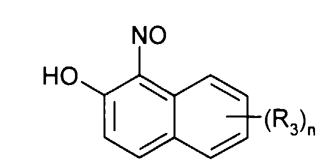 in which R₃ and n are as defined above, under basic conditions, such as those conditions obtained by the use of a tertiary amine for example, especially a tertiary amine such as triethylamine for example, to provide a compound of formula (I), in which A, R₁, R₂, R₃, R₄, n and m are as defined above.

This method of preparation constitutes another aspect of the presently claimed invention.

This method encompasses the preparation of compounds of formula (IV).

Said compounds of formula (IV) are obtained according to an original synthesis scheme whose various steps are known to the person skilled in the art, or are adapted from the literature. Said compounds of formula (IV) are also novel and thus constitute a further aspect of the present invention.

It is to the credit of the Applicant to have prepared and tested the original spiroindolinonaphthoxazine compounds of formula (I) described above; said compounds possess particularly advantageous photochromic properties. More specifically, these novel compounds possess a high colorability, with higher λmax values than the known N-methyl-substituted naphthospiroxazine compounds of analogous structure tested (Control).

Furthermore, these compounds are compatible with the organic polymer or inorganic material support matrices, both in the form included in said matrices and in the form of a coating of said matrices.

In solution or in a polymer matrix, the compounds according to the invention are colourless or faintly coloured in the initial sate and rapidly develop an intense coloration under UV light (365 nm) or a light source of the solar type. Finally, they regain their initial coloration when the irradiation ceases.

According to another of its objects, the present invention relates to the use of said compounds of formula (I) of the invention as photochromic agents. In other words, the Applicant presently proposes:
- novel photochromic compounds which consist of the spiroindolinonaphthoxazine derivatives such as defined above, taken alone or in a mixture of themselves and/or with at least one other photochromic compound of another type and/or with at least one non-photochromic colouring agent;
- novel photochromic compositions which comprise at least one spiroindolinonaphthoxazine derivative such as defined above and/or at least one (co)polymer and/or reticulate having at least one of said spiroindolinonaphthoxazine derivatives of the invention in its structure. Such photochromic compositions can contain at least one other photochromic compound, of another type and/or at least one non-photochromic colouring agent and/or at least one stabilising agent.

Said photochromic compounds of another type, non-photochromic colouring agents, and stabilising agents are prior art products known to the person skilled in the art.

Combinations of photochromic compounds of the invention and/or photochromic compounds of the invention and photochromic compounds of another type according to the prior art are particularly recommended which are suitable for generating grey or brown tints.

The compounds of the invention, notably as photochromic compounds, can be used in solution. Thus, a photochromic solution can be obtained by dissolving at least one of said compounds in an organic solvent such as toluene, dichloromethane, tetrahydrofuran or ethanol. The solutions obtained are in general colourless and transparent. When exposed to sunlight, they develop a high coloration and regain the colourless state when they are placed in an area of less exposure to the sun's rays or, in other words, when they are no longer submitted to UV. In general, a very low concentration of product (of the order of 0.01 to 5% by weight) is sufficient to obtain an intense coloration.

The compounds of the invention (spiroindolinonaphthoxazine derivatives of formula (I)) can also be used as a photochromic material dispersed uniformly in the mass or on the surface of a polymer matrix. In fact, the most interesting applications of the compounds of the invention are those in which the photochrome is dispersed uniformly within or on the surface of a polymer, copolymer or mixture of polymers. The (co)polymer matrix which comprises said photochrome of the invention (at least one, in a free form, and/or in the form of a (co)polymer and/or reticulate, and/or in the form of a photochromic composition, such as defined above) constitutes another object of the present invention.
The methods of implementation which can be envisaged in order to obtain such a matrix are very varied. Amongst those known to the person skilled in the art, the diffusion in the (co)polymer, from a suspension or solution of the photochrome, in a silicone oil, in an aliphatic or aromatic hydrocarbon, or in a glycol, or from another polymer matrix, can be cited for example. The diffusion is commonly carried out at a temperature of 50 to 200°C for a period of time of 15 minutes to several hours, according to the nature of the polymer matrix. Another implementation technique consists in mixing the photochrome in a formulation of polymerizable matrices, depositing this mixture on a surface or in a mould, and then carrying out the copolymerisation. These implementation techniques , and others, are described in the article by Crano *et al.* "Spiroxazines and their use in photochromic lenses" published in Applied Photochromic Polymer Systems, Ed. Blackie and Son Ltd - 1992.

In accordance with a variant of this object of the invention, it is also envisagable to graft the photochromes onto the (co)polymers. Thus, the invention also relates to the (co)polymers grafted by at least one of the photochromes described above. Thus, the expression "(co)polymer matrix comprising at least one photochrome of the invention" means both matrices which comprise said photochrome in their mass and on their surface, and matrices grafted by said photochrome.

The following products can be mentioned as examples of polymeric materials preferred for optical applications of the photochromic compounds according to the invention:
- optionally halogenated alkyl, cycloalkyl, aryl or aralkyl poly[mono-, di-, tri-, tetra-]acrylate or poly[mono-, di-, tri-, tetra-]methacrylate or having at least one ether and/or ester and/or carbonate and/or carbamate and/or thiocarbamate and/or urea and/or amide group,
- polystyrene, polyether, polyester, polycarbonate (e. g. bisphenol-A polycarbonate, diallyl diethylene glycol polycarbonate), polycarbamate, polyepoxy, polyurea, polyurethane, polythiourethane, polysiloxane, polyacrylonitrile, polyamide, aliphatic or aromatic polyester, vinylic polymers, cellulose acetate, cellulose triacetate, cellulose acetate-propionate or polyvinylbutyral,
- copolymers of at least two types of co-polymerizable monomers selected from precursor monomers of the polymers listed above (notably selected from (meth)acrylics, vinyls, allyls, and mixtures thereof).

Resins which are specifically described in French patent application No. 97 05458 of the 2^{nd} May 1997 (unpublished at present) may be especially mentioned.

The photochromic compounds according to the invention can be used alone or in a mixture with other products in order to form a composition which can be a solid or a liquid, in solution or in suspension for example, as has already been indicated above. These compositions, which constitute an object of the invention as already indicated above, can therefore comprise the compounds of the invention and other additional photochromic compounds enabling obtaining dark colourations, grey or brown for example, desired by the public in applications such as ophthalmic or solar spectacles trade. These additional photochromic compounds can be those known to the person skilled in the art and described in the literature, e. g. chromenes (US-A-3,567,605, US-A-5,238,981, WO-A-94 22850, EP-A 562 915), spiropyrans or naphthospiropyrans (US-A-5,238,981) and spiroxazines (Crano *et al.,* "Applied Photochromic Polymer Systems", Ed. Blackie & Son Ltd, 1992, chapter 2 ).

Said compositions according to the invention can also comprise :
- non-photochromic colouring agents which enable adjusting the tint,
- and/or one or more stabilising agents, such as an anti-oxidising agent for example,
- and/or one or more anti-UV,
- and/or one or more anti-radicals,
- and/or one or more photochemical excited state deactivators.

These additives can notably enable improving the durability of said compositions.

According to another of its aspects relative to the application of the compounds of the invention, another object of the present invention is ophthalmic articles, such as articles for the ophthalmic and solar spectacles trade, which comprise at least one compound according to the invention and/or at least one (co)polymer and/or reticulate formed, at least in part, from compound(s) of the invention and/or at least one composition containing at least one compound of the invention and/or at least one matrix, such as defined above, of an organic polymer material or an inorganic material or even of an inorganic-organic hybrid material incorporating therein at least one compound of the invention.

In practice, the articles most particularly covered by the present invention are photochromic ophthalmic or solar lenses, glazing (window panes for buildings, locomotion engines, automobiles), optical devices, decorative devices, solar protection devices, information storage, etc.

The present invention is illustrated by the following Examples of synthesis and photochromic validation, of the compounds of the invention (spiroindolinonaphthoxazines). Said compounds of the invention are compared to a prior art compound (Control).

### EXAMPLES

### GENERAL:

¹H NMR spectra were recorded in d₆-DMSO, CD₃CN and CDCl₃ on a Brüker AC-200p and Brüker AMX-400 spectrometers using tetramethylsilane as internal standard.

Mass spectra were obtained on a Varian MAT 311A mass spectrometer at an ionisation energy of 70 eV with a direct insertion of samples into the ionisation chamber.

The progress of the reactions was monitored by TLC on DC-Alufolien Kieselgel-60 F₂₅₄ plates (from Merck).

### Example 1: 3,3-dimethyl-1-[(N-phenyl)aminocarbonyltrimethylene]-spiro-[indolino-2,3'-[3H]-naphtho[2,1-b]-[1,4]oxazine].

Formula (I) : A = (CH₂)₃ , R₁ = H, R₂ = phenyl, m = n = 0.

Step 1a : 4-bromobutanoyl chloride.
   Thionyl chloride (0.88 ml, 1.43 g, 11.90 mmol) was added slowly to 4-bromobutanoic acid (1.0 g, 5.99 mmol). The resulting mixture was heated under reflux for 4 hours, and the excess thionyl chloride was then removed *in vacuo.* The crude 4-bromobutanoyl chloride was used as such in the next step without purification.
Step 1b : 4-bromobutanoic acid N-phenylamide.
   The crude 4-bromobutanoyl chloride prepared in step la above was dissolved in dry benzene and 1.11 g (5.99 mmol) of aniline hydrochloride was added thereto. The resulting reaction mixture was heated under reflux for 20 hours, cooled to room temperature, and the benzene was removed *in vacuo.* The resulting oil was then continuously extracted with hot heptane. The extracts were then allowed to cool and the precipitate formed was filtered and dried to give 4-bromobutanoic acid N-phenylamide (0.77 g, 53%).
   ¹H NMR spectrum (d₆-DMSO) : 1.80 (m, 2H, CH₂), 2.38 (m, 2H, CH₂CO), 7.02 (t, 1H, p-Ph, J = 7.8), 7.27 (t, 2H, m-Ph, J = 7.8), 7.58 (t, 2H, o-Ph, J = 7.8), 9.97 (s, 1H, NH). The CH₂Br signal overlaps the signal from H₂O contained in the DMSO.
Step 1c: 1-[3-(N-phenylaminocarbonyl)trimethylene]-2,3,3-trimethylindoleninium bromide.
   A mixture of 2,3,3-trimethylindolenine (239 mg, 1.5 mmol) and 4-bromobutanoic acid phenylamide prepared in step 1b above (399 mg, 1.65 mmol) was heated in an oil bath at 120-125°C under an argon atmosphere for 20 hours. The crude product was then purified by sequential extraction with hot benzene to give practically pure 1-[3-(N-phenylaminocarbonyl)trimethylene]-2,3,3-trimethylindoleninium bromide (463 mg, 77%) as a red-brown solid.
   ¹H NMR spectrum (d₆-DMSO): 1.53 (s, 6H, 2CH₃), 1.80 (m, 2H, CH₂), 2.38 (t, 2H, CH₂CO, J = 7.5), 2.88 (s, 3H, CH₃), 4.53 (t, 2H, CH₂N, J = 7.5), 7.01 (m, 1H, p-Ph), 7.30 (t, 2H, m-Ph, J = 7.8), 7.58 (t, 2H, o-Ph, J = 7.8), 7.62 (m, 2H, H-5, H-6), 7.81 (m, 1H, H-4), 8.02 (m, 1H, H-7), 9.97 (s, 1H, NH).
Step 1d : 2-hydroxy-1-nitrosonaphthalene.
   1-nitroso-2-hydroxynaphthalene was either obtained commercially or was prepared in the following manner :
   3.0 g (0.02 mol) of 2-hydroxynaphthalene was dissolved in 40 ml of 90% acetic acid. The resulting solution was then cooled to 0°C and a solution of 1.45 g (0.02 mol) of sodium nitrite in 35 ml of water was then added slowly. The reaction mixture was stirred for 2 hours at 0°C, and 100 ml of water were then added. The precipitate formed was filtered off and dried to give 1.96 g (54%) of 2-hydroxy-1-nitrosonaphthalene.
Step 1e: 3,3-dimethyl-1-[(N-phenyl)aminocarbonyltrimethylene]-spiro-[indolino-2,3'-[3H]-naphtho[2,1-b]-[1,4]oxazine].
   A mixture of 1-[3-(N-phenylaminocarbonyl)trimethylene]-2,3,3-trimethylindoleninium bromide (463 mg, 1.15 mmol), 2-hydroxy-1-nitrosonaphthalene (197 mg, 1.15 mmol), triethylamine (0.2 ml, 1.4 mmol) and absolute ethanol (5 ml) was heated under reflux for 3 hours under an argon atmosphere. The solvent was then removed *in vacuo,* and the residue was dissolved in benzene, and the triethylammonium bromide precipitate was filtered off. The filtrate was evaporated off *in vacuo* and the resulting oil was chromatographed on sylanised silica gel (for preparative TLC, Merck, eluent = benzene). The fractions containing the photochromic compound were combined, the solvent was then removed *in vacuo* to give 115 mg (21%) of 3,3-dimethyl-1-[(N-phenyl)aminocarbonyltrimethylene]-spiro-[indolino-2,3'-[3H]-naphtho[2,1-b]-[1,4]oxazine], as a brownish solid.
   ¹H NMR spectrum (d₆-DMSO): 1.26 (s, 3H, CH₃), 1.30 (s, 3H, CH₃), 1.96 (m, 2H, CH₂), 2.38 (t, 2H, CH₂ , J = 7.3), 3.20 (m, 2H, CH₂), 6.74 (d, 1H, H-4, J = 7.8), 6.83 (t, 1H, H-5, J = 7.3), 7.02 (t, 1H, p-Ph, J = 7.5), 7.15 (m, 3H, H-6, H-7, H-7'), 7.26 (t, 2H, m-Ph), 7.42 (m, 1H, H-8'), 7.55 (m, 3H, o-Ph, H-9'), 7.78 (d, 1H, H-5', J = 9.0), 7.84 (d, 1H, H-6', J = 8.1), 7.92 (s, 1H, H-1'), 8.45 (d, 1H, H-10', J = 8.4), 9.75 (s, 1H, NH).

### Example 2: 3,3-dimethyl-1-[(N-methyl-N-phenyl)aminocarbonylpentamethylene]-spiro-[indolino-2,3'-[3H]-naphtho[2,1-b]-[1,4]oxazine].

Formula (I): A = (CH₂)₅ , R₁ = CH₃ , R₂ = phenyl, m = n = 0.

Step 2a : 6-bromohexanoyl chloride.
   Thionyl chloride (0.38 ml, 0.61 g, 5.12 mmol) was added slowly to 6-bromohexanoic acid (0.5 g, 2.56 mmol). The resulting mixture was heated under reflux for 4 hours, and the excess thionyl chloride was then removed *in vacuo.* The crude 6-bromohexanoyl chloride was used as such in the next step without purification.
Step 2b : 6-bromohexanoic acid N-methyl-N-phenylamide.
   The crude 6-bromohexanoyl chloride prepared in step 2a above was dissolved in dry benzene and 0.41 g (2.82 mmol) of N-methylaniline hydrochloride was added thereto. The resulting reaction mixture was heated under reflux for 20 hours, cooled to room temperature, and the benzene was removed *in vacuo.* The resulting oil was then continuously extracted with hot hexane. The extracts were then allowed to cool and the precipitate formed was filtered and dried to give 6-bromohexanoic acid N-methyl-N-phenylamide (0.61 g, 83%).
   ¹H NMR spectrum (d₆-DMSO): 1.28 (m, 2H, CH₂), 1.49 (m, 2H, CH₂), 1.69 (m, 2H, CH₂), 2.06 (m, 2H, CH₂CO), 3.42 (m, 2H, CH₂Br), 4.03 (bs, 3H, N-CH₃), 7.29 (t, 2H, m-Ph, J = 7.8), 7.34 (t, 1H, p-Ph, J = 7.8), 7.44 (t, 2H, o-Ph, J = 7.8).
Step 2c : 1-[5-(N-methyl-N-phenylaminocarbonyl)pentamethylene]-2,3,3-trimethylindoleninium bromide.
   A mixture of 2,3,3-trimethylindolenine (234 mg, 1.47 mmol) and 6-bromohexanoic acid N-methyl-N-phenylamide prepared in step 2b above (457 mg, 1.61 mmol) was heated in an oil bath at 120-125°C under an argon atmosphere for 40 hours. The crude product was then purified by sequential extraction with hot hexane :benzene mixture (3 :1) to give practically pure 1-[5-(N-methyl-N-phenylaminocarbonyl)pentamethylene]-2,3,3-trimethylindoleninium bromide (652 mg, 100%) as a red-brown solid.
Step 2d : 3,3-dimethyl-1-[(N-methyl-N-phenyl)aminocarbonylpentamethylene]spiro-[indolino-2,3'-[3H]-naphtho[2,1-b]-[1,4]oxazine].
   A mixture of 1-[5-(N-methyl-N-phenylaminocarbonyl)pentamethylene]-2,3,3-trimethylindoleninium bromide (532 mg, 1.2 mmol), 2-hydroxy-1-nitrosonaphthalene (208 mg, 1.2 mmol), triethylamine (0.2 ml, 1.4 mmol) and absolute ethanol (5 ml) was heated under reflux for 3 hours under an argon atmosphere. The solvent was then removed *in vacuo,* and the residue was dissolved in benzene, and the residue was extracted with hot benzene. The filtrate was evaporated off *in vacuo* and the resulting oil was chromatographed on sylanised silica gel (for preparative TLC, Merck, eluents = benzene-hexane 1 :2 - 1 :1 (v/v), benzene). The fractions containing the photochromic compound were combined, the solvent was then removed *in vacuo* to give 107 mg (17%) of 3,3-dimethyl-1-[(N-methyl-N-phenyl)aminocarbonylpentamethylene]-spiro-[indolino-2,3'-[3H]-naphtho[2,1-b]-[1,4]oxazine], as a brown oil.
   ¹H NMR spectrum (d₆-DMSO): 1.15 (m, 2H, CH₂), 1.27 (s, 3H, CH₃), 1.35 (m, 2H, CH₂), 1.96 (m, 2H, CH₂), 3.02 (m, 2H, CH₂), 3.11 (s, 3H, NCH₃), 6.56 (d, 1H, H-4, J = 7.8), 6.81 (t, 1H, H-5, J = 7.3), 7.07 (d, 1H, H-7', J = 8.4), 7.17 (m, 3H, H-6, H-7, p-Ph), 7.22 (m, 2H, m-Ph), 7.40 (m, 3H, o-Ph, H-8'), 7.58 (m, 1H, H-9'), 7.78 (d, 1H, H-5', J = 8.7), 7.83 (s, 1H, H-1'), 7.85 (d, 1H, H-6', J = 8.4), 8.48 (d, 1H, H-10', J = 8.4).

### Example 3: 3,3-dimethyl-1-{[N-(4-trifluoromethylphenyl)]-aminocarbonylpentamethylene}-spiro-[indolino-2,3'-[3H]-naphtho[2,1-b]-[1,4]oxazine].

Formula (I): A = (CH₂)₅ , R₁ = H , R₂ = 4-(trifluoromethyl)phenyl, m = n = 0.

Step 3a : 6-bromohexanoyl chloride.
   6-bromohexanoyl chloride was prepared as in step 2a above.
Step 3b : 6-bromohexanoic acid 4-(trifluoromethyl)phenylamide.
   The 6-bromohexanoyl chloride prepared in step 2a above was dissolved in dry benzene and 0.56 g (2.82 mmol) of p-trifluoromethylaniline hydrochloride was added thereto. The resulting reaction mixture was heated under reflux for 40 hours, cooled to room temperature, and the precipitate formed was filtered off, washed with benzene and water and dried to give 0.49 g (56%) of 6-bromohexanoic acid 4-(trifluoromethyl)phenylamide.
   ¹H NMR spectrum (d₆-DMSO): 1.44 (m, 2H, CH₂), 1.63 (m, 2H, CH₂), 2.36 (m, 2H, CH₂CO), 7.64 (t, 2H, o-Ph, J = 8.4), 7.80 (t, 2H, m-Ph, J = 8.4), 10.19 (bs, 1H, NH). The CH₂Br signal overlaps the signal from H₂O contained in the DMSO.
Step 3c : 1-[(4-trifluoromethylphenyl)aminocarbonylpentamethylene]-2,3,3-trimethyl-indoleninium bromide.
   A mixture of 2,3,3-trimethylindolenine (191 mg, 1.2 mmol) and 6-bromohexanoic acid 4-(trifluoromethyl)phenylamide prepared in step 3b above (450 mg, 1.33 mmol) was heated in an oil bath at 120-125°C under an argon atmosphere for 40 hours. The crude product was then purified by sequential extraction with hot hexane :benzene mixture (4 :1) to give 797 mg (100%) of practically pure 1-[(4-trifluoromethylphenyl)aminocarbonylpentamethylene]-2,3,3-trimethylindoleninium bromide as a red-brown solid.
   ¹H NMR spectrum (d₆-DMSO) : 1.47 (m, 2H, CH₂), 1.53 (s, 6H, CH₂), 1.67 (m, 2H, CH₂), 1.89 (m, 2H, CH₂), 2.40 (t, 2H, CH₂CO, J = 7.5), 2.84 (s, 3H, CH₃), 4.48 (t, 2H, CH₂N, J = 7.5), 7.62 (m, 4H, H-5, H-6, o-Ph), 7.81 (m, 3H, H-4, m-Ph), 7.97 (m, 1H, H-7), 10.26 (s, 1H, NH).
Step 3d : 3,3-dimethyl-1-{[N-(4-trifluoromethylphenyl)]aminocarbonylpentamethylene}-spiro-[indolino-2,3'-[3H]-naphtho[2,1-b]-[1,4]oxazine].
   A mixture of 1-[(4-trifluoromethylphenyl)aminocarbonylpentamethylene]-2,3,3-trimethyl-indoleninium bromide (720 mg, 1.45 mmol), 2-hydroxy-1-nitrosonaphthalene (249 mg, 1.45 mmol), triethylamine (0.25 ml, 1.8 mmol) and absolute ethanol (10 ml) was heated under reflux for 3 hours under an argon atmosphere. The solvent was then removed *in vacuo,* and the residue was dissolved in benzene, the precipitate of triethylammonium bromide was then filtered off. The filtrate was evaporated off *in vacuo* and the resulting oil was chromatographed on sylanised silica gel (for preparative TLC, Merck, eluents = benzene-hexane 1 :2 - 1 :1 (v/v), benzene). The fractions containing the photochromic compound were combined, the solvent was then removed *in vacuo* to give 220 mg (27%) of 3,3-dimethyl-1-{[N-(4-trifluoromethylphenyl)]aminocarbonylpentamethylene}-spiro-[indolino-2,3'-[3H]-naphtho[2,1-b]-[1,4]oxazine] as an emerald-green solid.
   ¹H NMR spectrum (d₆-DMSO): 1.25-1.40 (m, 10H, 2CH₃ + 2CH₂), 1.50 (m, 2H, CH₂), 2.27 (m, 2H, CH₂), 3.16 (m, 2H, CH₂), 6.61 (d, 1H, H-4, J = 7.8), 6.80 (t, 1H, H-5, J = 7.3), 7.15 (m, 3H, H-6, H-7, H-7'), 7.40 (m, 1H, H-8'), 7.56 (m, 1H, H-9'), 7.63 (m, 2H, 2H-Ph), 7.78 (m, 3H, 2H-Ph, H-5'), 7.83 (d, 1H, H-6', J = 8.1), 7.87 (s, 1H, H-1'), 8.45 (d, 1H, H-10', J = 8.4), 10.11 (s, 1H, NH).

### Example 4: 3,3-dimethyl-1-{[N-(4-methoxyphenyl)]aminocarbonylpentamethylene}-spiro-[indolino-2,3'-[3H]-naphtho[2,1-b]-[1,4]oxazine].

Formula (I) : A = (CH₂)₅ , R₁ = H , R₂ = 4-methoxyphenyl, m = n = 0.

Step 4a : 6-bromohexanoyl chloride.
   Thionyl chloride (0.75 ml, 1.23 g, 10.30 mmol) was added slowly to 6-bromohexanoic acid (1.00 g, 5.13 mmol). The resulting mixture was heated under reflux for 4 hours, and the excess thionyl chloride was then removed *in vacuo.* The crude 6-bromohexanoyl chloride was used as such in the next step without purification.
Step 4b : 6-bromohexanoic acid 4-(methoxy)phenylamide.
   The crude 6-bromohexanoyl chloride prepared in step 4a above was dissolved in dry benzene and 1.095 g (5.13 mmol) of p-methoxyaniline hydrochloride was added thereto. The resulting reaction mixture was heated under reflux for 20 hours, cooled to room temperature, and the benzene was removed in vacuo. The resulting oil was continuously extracted with hot heptane. The extracts were then cooled and the precipitate formed was filtered off and dried to give 1.141 g (74%) of 6-bromohexanoic acid 4-(methoxy)phenylamide.
   ¹H NMR spectrum (d₆-DMSO) : 1.44 (m, 2H, CH₂), 1.60 (m, 2H, CH₂), 1.84 (m, 2H, CH₂), 2.25 (m, 2H, CH₂CO), 3.52 (m, 2H, CH₂Br), 6.86 (t, 2H, o-Ph, J=8,1), 7.48 (t,2H, m-Ph, J=8.1), 9.63 (bs, 1H, NH).
Step 4c : 1-[(4-methoxyphenyl)aminocarbonylpentamethylene]-2,3,3-trimethylindoleninium bromide.
   A mixture of 2,3,3-trimethylindolenine (238 mg, 1.5 mmol) and 6-bromohexanoic acid 4-(methoxy)phenylamide prepared in step 4b above (495 mg, 1.65 mmol) was heated in an oil bath at 120-125°C under an argon atmosphere for 40 hours. The crude product was then purified by sequential extraction with hot hexane :benzene mixture (3 :1) to give 687 mg (100%) of practically pure 1-[(4-methoxyphenyl)aminocarbonylpentamethylene]-2,3,3-trimethylindoleninium bromide as a red-brown solid.
   ¹H NMR spectrum (d₆-DMSO): 1.46 (m, 2H, CH₂), 1.52 (s, 6H, 2CH₃), 1.67 (m, 2H, CH₂), 1.87 (m, 2H, CH₂), 2.39 (t, 2H, CH₂CO, J = 7.6), 2.83 (s, 3H, CH₃), 4.47 (t, 2H, CH₂N, J = 7.6), 6.88 (d, 2H, o-Ph, J=9.3), 7.51 (d, 2H, m-Ph, J=9.3), 7.62 (m, 2H, H-5, H-6), 7.83 (m, 1H, H-4), 7.99 (m, 1H, H-7), 9.62 (s, 1H, NH).
Step 4d: 3,3-dimethyl-1-{[N-(4-methoxyphenyl)]aminocarbonylpentamethylene}-spiro-[indolino-2,3'-[3H]-naphtho[2,1-b]-[1,4]oxazine].
   A mixture of 1-[(4-methoxyphenyl)aminocarbonylpentamethylene]-2,3,3-trimethyl-indoleninium bromide (688 mg, 1.5 mmol), 2-hydroxy-1-nitrosonaphthalene (260 mg, 1.5 mmol), triethylamine (0.25 ml, 1.8 mmol) and absolute ethanol (10 ml) was heated under reflux for 3 hours under an argon atmosphere. The solvent was then removed *in vacuo,* and the residue was dissolved in benzene, the precipitate of triethylammonium bromide was then filtered off. The filtrate was evaporated off *in vacuo* and the resulting oil was chromatographed on sylanised silica gel (for preparative TLC, Merck, eluents = benzene-hexane 1 :1 (v/v), benzene). The fractions containing the photochromic compound were combined, the solvent was then removed *in vacuo* to give 106 mg (13%) of 3,3-dimethyl-1-{[(N-(4-methoxyphenyl)]aminocarbonylpentamethylene}-spiro-[indolino-2,3'-[3H]-naphtho[2,1-b]-[1,4]oxazine] as a green solid.
   ¹H NMR spectrum (d₆-DMSO): 1.20-1.30 (m, 8H, 2CH₃ + CH₂), 1.56 (m, 2H, CH₂), 2.22 (m, 2H, CH₂), 3.14 (m, 2H, CH₂), 3.71 (s, 3H, OCH₃), 6.63 (d, 1H, H-4, J = 7.8), 6.85 (m, 3H, H-6, H-7, H-7'), 7.10 (m, 3H, H-5, 2H-Ph), 7.45 (m, 3H, H-8', 2H-Ph), 7.57 (m, 1H, H-9'), 7.78 (d, 1H, H-5', J = 8.9), 7.84 (d, 1H, H-6', J = 8.1), 7.89 (s, 1H, H-1'), 8.48 (d, 1H, H-10', J = 8.4), 9.60 (s, 1H, NH). The CH₂CO signal overlaps the signal from H₂O contained in the DMSO.

### Example 5: 3,3-dimethyl-1-{[N-(4-nitrophenyl)]aminocarbonylpentamethylene}-spiro-[indolino-2,3'-[3H]-naphtho[2,1-b]-[1,4]oxazine].

Formula (I): A = (CH₂)₅ , R₁ = H , R₂ = 4-nitrophenyl, m = n = 0.

Step 5a : 6-bromohexanoyl chloride.
   Thionyl chloride (0.77 ml, 1.25 g, 10.49 mmol) was added slowly to 6-bromohexanoic acid (1.02 g, 5.25 mmol). The resulting mixture was heated under reflux for 10 hours, and the excess thionyl chloride was then removed *in vacuo.* The crude 6-bromohexanoyl chloride was used as such in the next step without purification.
Step 5b : 6-bromohexanoic acid 4-(nitro)phenylamide.
   The crude 6-bromohexanoyl chloride prepared in step 5a above was dissolved in dry benzene and 0.72 g (5.22 mmol) of p-nitroaniline was added thereto. The resulting reaction mixture was heated under reflux for 90 hours, cooled to room temperature, and the benzene was removed *in vacuo.* The resulting oil was continuously extracted with hot heptane. The extracts were then cooled and the precipitate formed was filtered off and dried to give 0.97 g (59%) of 6-bromohexanoic acid 4-(nitro)phenylamide.
   ¹H NMR spectrum (d₆-DMSO): 1.44 (m, 2H, CH₂), 1.63 (m, 2H, CH₂), 1.84 (m, 2H, CH₂), 2.40 (m, 2H, CH₂CO), 3.55 (m, 2H, CH₂Br), 7.84 (t, 2H, o-Ph, J=9.1), 8.22 (t,2H, m-Ph, J=9.1), 10.52 (bs, 1H, NH).
Step 5c : 1-[(4-nitrophenyl)aminocarbonylpentamethylene]-2,3,3-trimethyl-indoleninium bromide.
   A mixture of 2,3,3-trimethylindolenine (239 mg, 1.5 mmol) and 6-bromohexanoic acid 4-(nitro)phenylamide prepared in step 4b above (520 mg, 1.65 mmol) was heated in an oil bath at 120-125°C under an argon atmosphere for 40 hours. The crude product was then purified by sequential extraction with hot benzene to give 676 mg (95%) of practically pure 1-[(4-nitrophenyl)amino-carbonylpentamethylene]-2,3,3-trimethylindoleninium bromide as a red-brown solid.
   ¹H NMR spectrum (d₆-DMSO): 1.47 (m, 2H, CH₂), 1.53 (s, 6H, 2CH₃), 1.68 (m, 2H, CH₂), 1.88 (m, 2H, CH₂), 2.44 (t, 2H, CH₂CO, J = 7.6), 2.84 (s, 3H, CH₃), 4.49 (t, 2H, CH₂N, J = 7.6), 7.62 (m, 2H, H-5, H-6), 7.84 (m, 1H, H-4), 7.87 (d, 2H, o-Ph, J=9.3), 7.99 (m, 1H, H-7), 7.99 (m, 1H, H-7), 8.22 (d, 2H, m-Ph, J=9.3), 10.61 (s, 1H, NH).
Step 5d: 3,3-dimethyl-1-{[N-(4-nitrophenyl)]aminocarbonylpentamethylene}-spiro-[indolino-2,3'-[3H]-naphtho[2,1-b]-[1,4]oxazine].
   A mixture of 1-[(4-nitrophenyl)aminocarbonylpentamethylene]-2,3,3-trimethyl-indoleninium bromide (616 mg, 1.43 mmol), 2-hydroxy-1-nitrosonaphthalene (247 mg, 1.43 mmol), triethylamine (0.25 ml, 1.8 mmol) and absolute ethanol (10 ml) was heated under reflux for 3 hours under an argon atmosphere. The solvent was then removed *in vacuo,* and the residue was dissolved in benzene, the precipitate of triethylammonium bromide was then filtered off. The filtrate was evaporated off *in vacuo* and the resulting oil was chromatographed on sylanised silica gel (for preparative TLC, Merck, eluents = benzene-hexane 1 :1 - 1.5 :1 (v/v)). The fractions containing the photochromic compound were combined, the solvent was then removed *in vacuo* to give 179 mg (23%) of 3,3-dimethyl-1-{[N-(4-nitrophenyl)]aminocarbonylpentamethylene}-spiro-[indolino-2,3'-[3H]-naphtho[2,1-b]-[1,4]oxazine] as a green-blue solid.
   ¹H NMR spectrum (d₆-DMSO): 1.07 (m, 2H, CH₂), 1.21 (s, 3H, CH₃), 1.24 (s, 3H, CH₃), 1.57 (m, 2H, CH₂), 2.32 (m, 2H, CH₂), 3.11 (m, 2H, CH₂), 6.61 (d, 1H, H-4, J = 7.8), 6.79 (t, 1H, H-5, J = 7.3), 7.09 (m, 3H, H-6, H-7, H-7'), 7.39 (m, 1H, H-8'), 7.55 (m, 1H, H-9'), 7.78 (m, 4H, 2H-Ph, H-5', H-6'), 7.88 (s, 1H, H-1'), 8.18 (m, 2H, 2H-Ph), 8.44 (d, 1H, H-10', J = 8.4), 10.47 (s, 1H, NH).

The following compounds were also prepared by methods analogous to those described above in Examples 1 to 5 :

| Example | A | R₁ | R₂ | n | m |
|---|---|---|---|---|---|
| 6 | (CH₂)₅ | H | H | 0 | 0 |
| 7 | (CH₂)₇ | H | phenyl | 0 | 0 |
| 8 | (CH₂)₄ | H | phenyl | 0 | 0 |
| 9 | (CH₂)₅ | H | phenyl | 0 | 0 |
| 10 (not part of the invention) | (CH₂)₂ | H | H | 0 | 0 |

### Example 11 : 3,3-dimethyl-6'-morpholino-1-[(N-phenyl)aminocarbonylpentamethylene]-spiro-[indolino-2,3'-[3H]-naphtho[2,1-b]-[1,4]oxazine].

Formula (I) : A = (CH₂)₅ , R₁ = H , R₂ = phenyl, R₃ = 6'-morpholino, m = 0.

Step 11a: 6-bromohexanoyl chloride.
   Thionyl chloride (0.35 ml, 0.6 g, 5 mmol) was added slowly to 6-bromohexanoic acid (0.5 g, 2.5 mmol). The resulting mixture was heated under reflux for 4 hours, and the excess thionyl chloride was then removed *in vacuo.* The crude 6-bromohexanoyl chloride was used as such in the next step without purification.
Step 11b : 6-bromohexanoic acid phenylamide.
   The crude 6-bromohexanoyl chloride prepared in step 11a above was dissolved in dry benzene and 0.37 g (5.22 mmol) of aniline hydrochloride was added thereto. The resulting reaction mixture was heated under reflux for 20 hours, cooled to room temperature, and the formed was filtered off, washed with benzene and water, and dried to give 0.37 g (53%) of 6-bromohexanoic acid phenylamide.
   ¹H NMR spectrum (d₆-DMSO) : 1.53 (m, 2H, CH₂), 1.72 (m, 2H, CH₂), 1.93 (m, 2H, CH₂), 2.41 (m, 2H, CH₂CO), 7.11 (t, 1H, p-Ph, J=7.8), 7.38 (t, 2H, m-Ph, J=7.8), 7.68 (t, 2H, o-Ph, J=7.8), 9.93 (bs, 1H, NH). The CH₂Br signal ovelaps the signal of water contained in DMSO.
Step 11c: 1-[(N-phenyl)aminocarbonylpentamethylene]-2,3,3-trimethyl-indoleninium bromide.
   A mixture of 2,3,3-trimethylindolenine (135 mg, 0.85 mmol) and 6-bromohexanoic acid phenylamide prepared in step 11b above (253 mg, 0.94 mmol) was heated in an oil bath at 120-125°C under an argon atmosphere for 20 hours. The crude product was then purified by sequential extraction with hot benzene to give 300 mg (82%) of practically pure 1-[(N-phenyl)aminocarbonylpentamethylene]-2,3,3-trimethylindoleninium bromide as a red-brown solid.
   ¹H NMR spectrum (d₆-DMSO): 1.55 (m, 2H, CH₂), 1.62 (s, 6H, 2CH₃), 1.72 (m, 2H, CH₂), 1.97 (m, 2H, CH₂), 2.41 (t, 2H, CH₂CO), 2.93 (s, 3H, CH₃), 4.60 (t, 2H, CH₂N, J = 7.6), 7.11 (t, 1H, p-Ph, 7.8), 7.37 (t, 2H, m-Ph, J=7.8), 7.67 (d, 2H, o-Ph, J=7.8), 7.70 (m, 2H, H-5, H-6), 7.93 (m, 1H, H-4), 8.07 (m, 1H, H-7), 10.00 (s, 1H, NH).
Step 11d : 3,3-dimethyl-6'-morpholino-1-[(N-phenyl)aminocarbonylpentamethylene]-spiro-[indolino-2,3'-[3H]-naphtho[2,1-b]-[1,4]oxazine].
   A solution of 173 mg (1 mmol) of 2-hydroxy-1-nitrosonaphthalene in 3 ml of methanol was heated to reflux under argon. 0.18 ml (2 mmol) of morpholine was then added thereto. The resulting mixture was the heated under reflux for 1 hour, and 429 mg (1 mmol) of 1-[(N-phenyl)aminocarbonylpentamethylene]-2,3,3-trimethyl-indoleninium bromide, prepared in the preceding step, together with 0.17 ml (1.2 mmol) of triethylamine were then added thereto. The mixture was then heated under reflux for a further 3 hours. The solvent was then removed *in vacuo,* and the residue extracted with hot benzene. The filtrate was evaporated *in vacuo* and the resulting oil was chromatographed on sylanised silica gel (for preparative TLC, Merck, eluent - hexane-ethyl acetate, 50:1 - 10:1). The fractions containing the photochromic compound were then combined and the solvent removed *in vacuo* to give 130 mg (22 %) of 3,3-dimethyl-6'-morpholino-1-[(N-phenyl)aminocarbonylpentamethylene]-spiro-[indolino-2,3'-[3H]-naphtho-[2,1-b]-[1,4]oxazine], as a deep purple solid.

### Example 12: Preparation of photochromic matrices and lens samples containing the photochromic compounds of the invention.

The matrix used in accordance with the present invention may be one of two types:
I. MATRIX I:
   A homopolymer of the monomer DIACRYL 121 (D121 : tetraethoxylated Bisphenol A dimethylmethacrylate), marketed by Akzo Nobel ; or
II. MATRIX II:
   A copolymer comprised of a 50/50 mixture by weight of D121 (as above) and polyethylene glycol dimethacrylate (Molecular weight = 400) (PEGDMA 400).

The lens samples are prepared as follows :
10⁻⁵ moles of at least one compound of the invention, together with 0.2% by weight of AMBN (2,2'-azobis(2-methylbutyronitrile) provided by Akzo, are added to 10 g of formulations able to generate cross-linked matrix I or II.

The formulations thus prepared are then poured into a lens mould of 2 mm thickness and then cured by heating at 60°C for 6 hours, followed by heating at 80°C for 2 hours to give a lens sample.

The photochromic properties of the lens samples thus obtained were then evaluated as follows :
Said lens sample, containing said photochromic compounds in its mass, is exposed to a UV radiation (source: xenon lamp). The λₘₐₓ values in the visible and the discoloration kinetics are given in the Table I below :

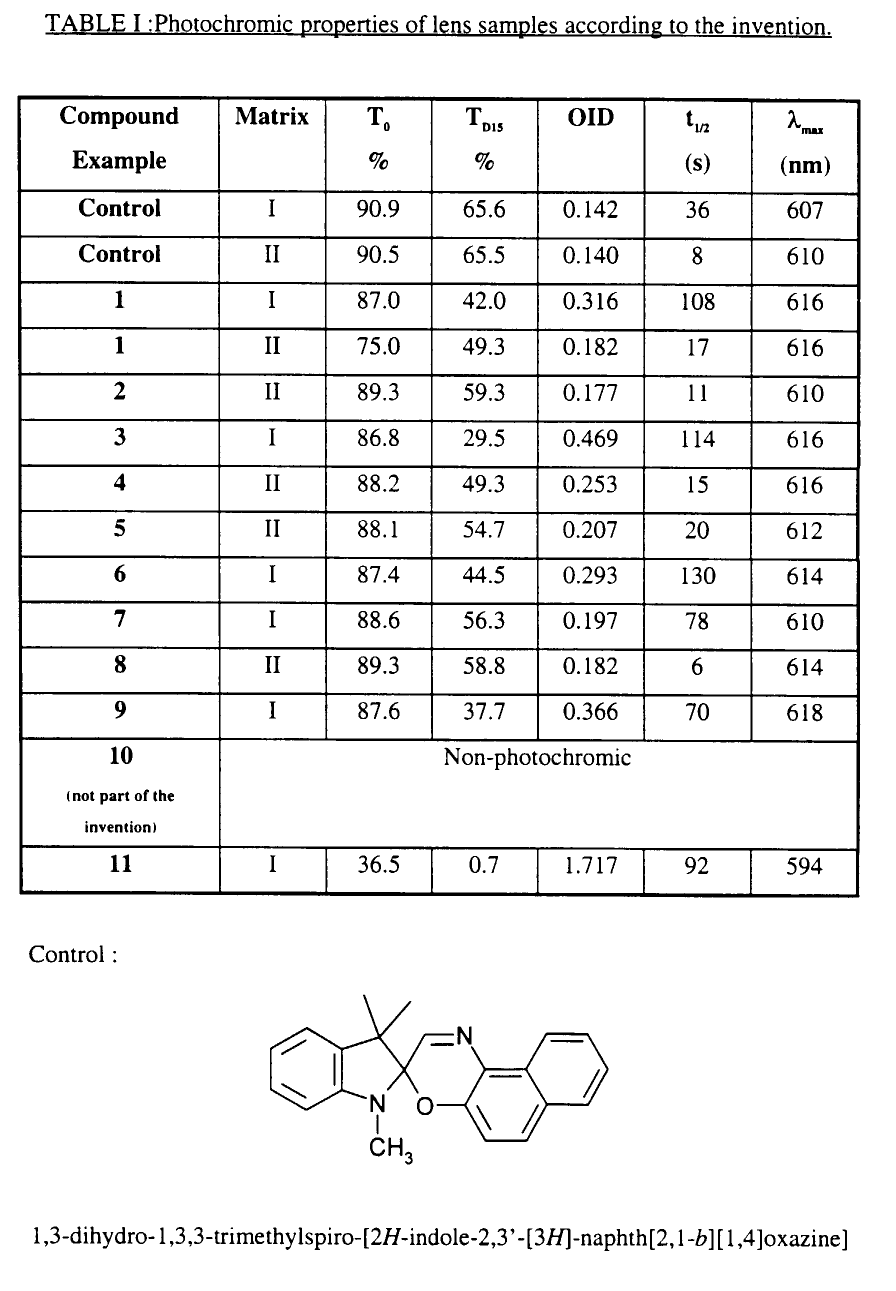

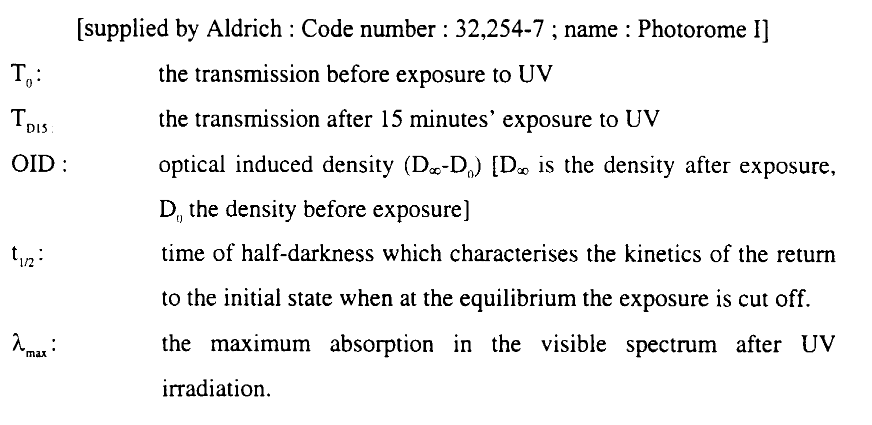

It is clearly seen from the above Table that when the lens samples containing photochromic compounds of the invention (having an N-arylamidoalkyl substituent on the N-atom of the 2,3,3-trimethylindoleninyl moiety of the molecule) are compared to the control lens samples (Control 1 and Control 2) containing photochromic compounds of the prior art (having not an N-arylamidoalkyl substituent on the N-atom of the 2,3,3-trimethylindoleninyl moiety of the molecule but simply a methyl group), the following may be noted :
i) the lens samples containing photochromic compounds of the invention have higher λₘₐₓ values than those containing prior art photochromic compound ;
ii) the lens samples containing photochromic compounds of the invention have higher optical induced densities (OID) than those containing prior art photochromic compound;
iii) the lens samples containing photochromic compounds of the invention have lower transmissions after 15 minutes' exposure to UV (T_{D15}) than those containing prior art photochromic compound.

## Claims

1. Compounds of formula (I): 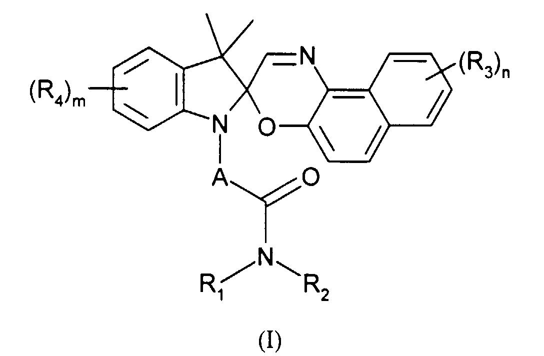 in which :
• A represents :
- a linear alkyl group having 3 to 12 carbon atoms, advantageously from 3 to 7 carbon atoms, or
- a branched alkyl group-containing alkyl group having 4 to 12 carbon atoms of formula :
―(CH₂)ᵣ―X―(CH₂)ₛ―
in which r is equal to or greater than 1, s is equal to or greater than 1, and X represents said branched alkyl group ;
• R₁ and R₂, identical or different, independently represent :
- hydrogen,
- a linear alkyl group having 1 to 4 carbon atoms, advantageously 1 or 2 carbon atoms,
- a cycloalkyl group having 3 to 12 carbon atoms,
- an aryl or aryloxy group having 6 to 24 carbon atoms in its basic structure, or a heteroaryl or heteroaryloxy group having 4 to 24 carbon atoms in its basic structure and at least one heteroatom selected from sulphur, oxygen and nitrogen ; said basic structures optionally being substituted with at least one substituent selected from :
+ a halogen and notably fluorine, chlorine and bromine,
+ a linear or branched alkyl group having 1 to 6 carbon atoms,
+ a linear or branched alkoxy group having 1 to 6 carbon atoms,
+ a linear or branched haloalkyl or haloalkoxy group having 1 to 6 carbon atoms, and notably a fluoroalkyl group of this type, advantageously a trifluoromethyl group,
+ a linear or branched alkenyl or alkynyl group having 2 to 12 carbon atoms and notably a vinyl group or an allyl group,
+ an -NO₂ group,
+ an -NH₂ group,
+ an -NHR group, R representing a linear or branched alkyl group having 1 to 6 carbon atoms,
+ a 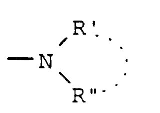 group, R' and R", identical or different, representing independently a linear or branched alkyl group having 1 to 6 carbon atoms or representing, together with the nitrogen atom to which they are bound, a 5- to 7-membered ring which can comprise at least one other heteroatom selected from oxygen, sulphur and nitrogen, said 5- to 7-membered ring being non-substituted or substituted with an R"' group, said R"' group being selected from a linear or branched alkyl group having 1 to 6 carbon atoms, an amine group, and a nitro group ; in the case in which said other heteroatom is nitrogen, said nitrogen may optionally be substituted with a linear or branched alkyl group having 1 to 6 carbon atoms,
+ a methacryloyl group or an acryloyl group,
+ an epoxy group of formula : 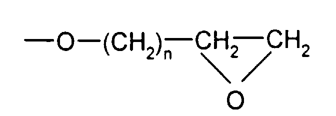 in which n = 1, 2 or 3,
- an aralkyl, aryloxyalkyl, heteroaralkyl or heteroaryloxyalkyl group, the linear or branched alkyl group having 1 to 4 carbon atoms and the aryl, aryolxy, heteroaryl, and heteroaryloxy groups having the definitions given above,
or
- said two substituents R₁ and R₂ together form with the N-atom to which they are bound, a 5- to 7-membered ring which can comprise at least one other heteroatom selected from oxygen, sulphur and nitrogen, said 5- to 7-membered ring being non-substituted or substituted with an R"' group, said R"' group being selected from a linear or branched alkyl group having 1 to 6 carbon atoms, an amine group, and a nitro group ; in the case in which said other heteroatom is nitrogen, said nitrogen may optionally be substituted with a linear or branched alkyl group having 1 to 6 carbon atoms ; said two substituents R₁ and R₂ , together with the N-atom to which they are bound, representing advantageously a morpholino, piperidino, piperazino, or an indolino group ;
• R₃ and R₄ , identical or different, independently represent :
- a halogen, and notably fluorine, chlorine or bromine,
- a linear or branched alkyl group having 1 to 12 carbon atoms (advantageously 1 to 6 carbon atoms),
- a cycloalkyl group having 3 to 12 carbon atoms,
- a linear or branched alkenyl or alkynyl group having 2 to 12 carbon atoms,
- a linear or branched alkoxy or alkylthio group, having 1 to 12 carbon atoms (advantageously 1 to 6 carbon atoms),
- a haloalkyl, a halocycloalkyl or haloalkoxy group corresponding respectively to the alkyl, cycloalkyl, alkoxy groups above, substituted with at least one halogen atom, notably selected from fluorine, chlorine and bromine,
- an electron-withdrawing group, such as a CN, NO₂ or SCN group,
- an aryl, aryloxy, heteroaryl or heteroaryloxy group having the same definition as that given above for R₁, R₂,
- an aralkyl, aryloxyalkyl, heteroaralkyl or heteroaryloxyalkyl group, the linear or branched alkyl group having 1 to 4 carbon atoms and the aryl, aryloxy, heteroaryl, and heteroaryloxy groups having the same definitions as those given above for R₁, R₂,
- an amino or amido group: -NH₂, - NHR, - CONH₂, - CONHR, 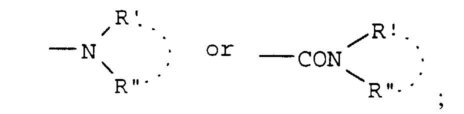 R, R', R" having their respective definitions given above for the amine substituents of the R₁, R₂ values: aryl or heteroaryl,
- an - OCOR₆ or -COOR₆ group, R₆ representing a hydrogen atom, or a straight or branched alkyl group having 1 to 6 carbon atoms or a cycloalkyl group having 3 to 6 carbon atoms or a phenyl group optionally substituted with at least one of the substituents listed above for the R₁, R₂ values: aryl, aryloxy, heteroaryl or heteroaryloxy ; or
- in the case in which m is equal to or greater than 2 in (R₄)ₘ , and/or in the case in which n is equal to or greater than 2 in (R₃)ₙ, said radicals, being preferably borne by two adjacent carbon atoms, can combine to form at least one aromatic ring having 5 or 6 members, or an aliphatic ring having 5 to 7 members, preferably 5 or 6 members, said ring(s) comprising, optionally, at least one heteroatom selected from sulphur, oxygen and nitrogen, so as to form at least one heterocyclic ring, the latter being optionally substituted by one or more radicals, which may be identical or different, and have the same definition as given above for R₄ and R₃ ;
• m is an integer of 0 to 4 ; and
• n is an integer of 0 to 6.

2. Compounds according to claim 1 of formula (I') : 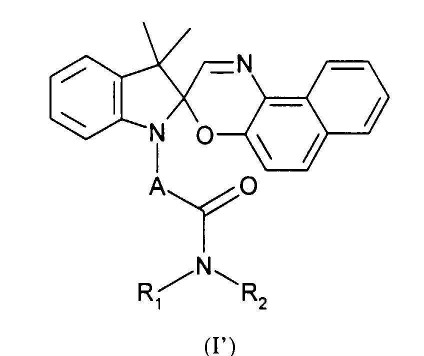 in which A, R₁ and R₂ are as defined in claim 1 with reference to formula (I).

3. Compounds according to claim 1 or 2 of formula (I) or (I') in which one of R₁ and R₂ is an unsubstituted or substituted aryl group, advantageously an unsubstituted or substituted phenyl group.

4. Compounds selected from the group consisting of the following :
3,3-dimethyl-1-[(N-phenyl)aminocarbonyltrimethylene]-spiro-[indolino-2,3'-[3H]-naphtho[2,1-b]-[1,4]oxazine] : 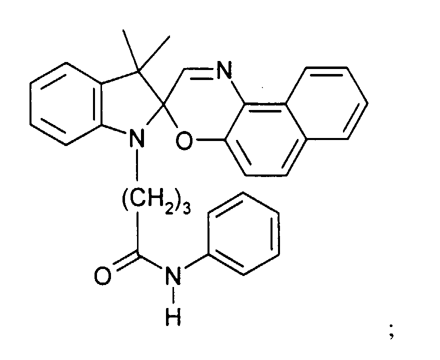
3,3-dimethyl-1-[(N-methyl-N-phenyl)aminocarbonylpentamethylene]-spiro-[indolino-2,3'-[3H]-naphtho[2,1-b]-[1,4]oxazine] : 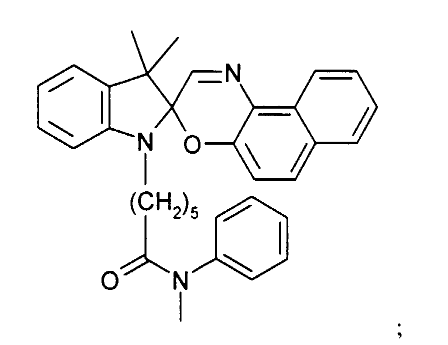
3,3-dimethyl-1-{[N-(4-trifluoromethylphenyl)]aminocarbonylpentamethylene}-spiro-[indolino-2,3'-[3H]-naphtho[2,1-b]-[1,4]oxazine] : 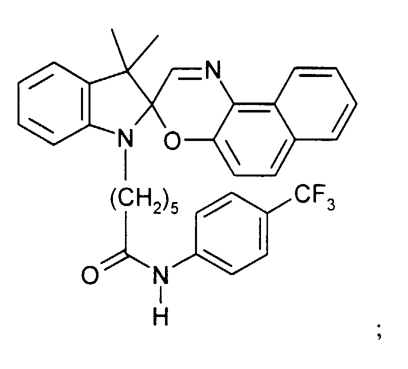
3,3-dimethyl-1-{[N-(4-methoxyphenyl)]aminocarbonylpentamethylene}-spiro-[indolino-2,3'-[3H]-naphtho[2,1-b]-[1,4]oxazine] : 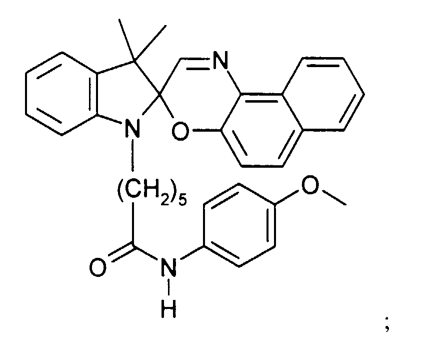
3,3-dimethyl-1-{[N-(4-nitrophenyl)]aminocarbonylpentamethylene}-spiro-[indolino-2,3'-[3H]-naphtho[2,1-b]-[1,4]oxazine] : 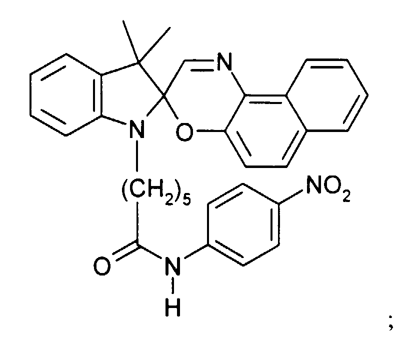
3,3-dimethyl-6'-morpholino-1-[(N-phenyl)aminocarbonylpentamethylene]-spiro-[indolino-2,3'-[3H]-naphtho[2,1-b]-[1,4]oxazine] : 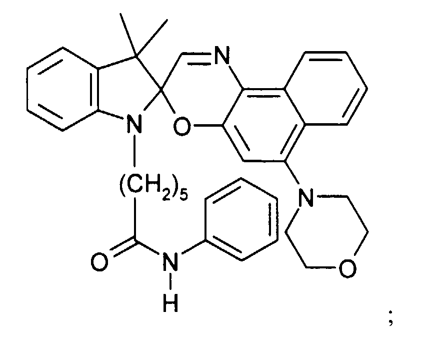
1-(aminocarbonylpentamethylene)-3,3-dimethyl-spiro-[indolino-2,3'-[3H]-naphtho[2,1-b]-[1,4]oxazine] : 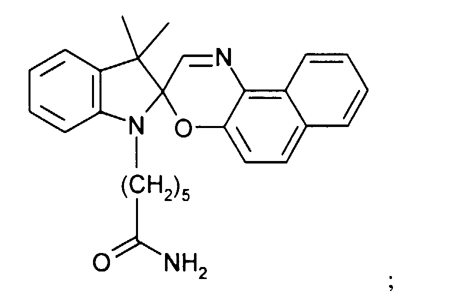
3,3-dimethyl-1-[(N-phenyl)aminocarbonylheptamethylene]-spiro-[indolino-2,3'-[3H]-naphtho[2,1-b]-[1,4]oxazine] : 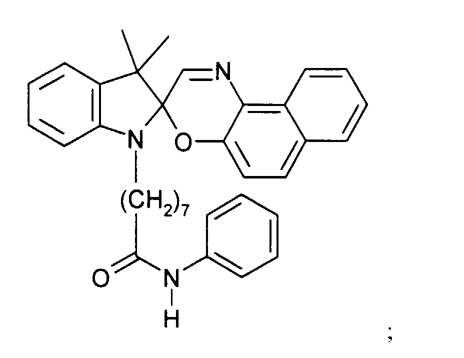
3,3-dimethyl-1-[(N-phenyl)aminocarbonyltetramethylene]-spiro-[indolino-2,3'-[3H]-naphtho[2,1-b]-[1,4]oxazine] : 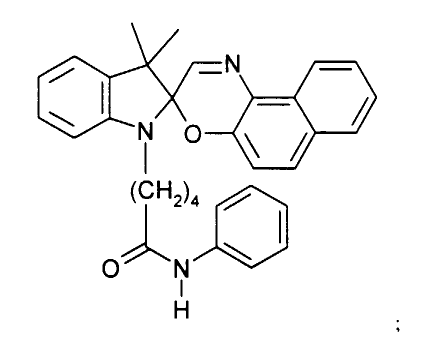
3,3-dimethyl-1-[(N-phenyl)aminocarbonylpentamethylene]-spiro-[indolino-2,3'-[3H]-naphtho[2,1-b]-[1,4]oxazine] :

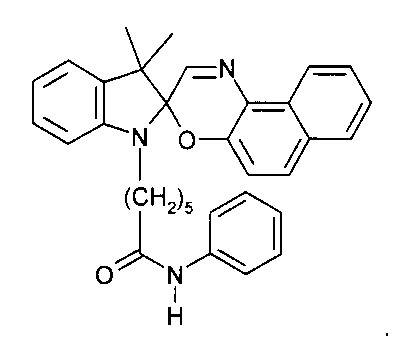

5. A method of preparing compounds of formula (I) according to any one of claims 1 to 4, characterised in that it comprises :
a) allowing an indolenine derivative of formula (II) : 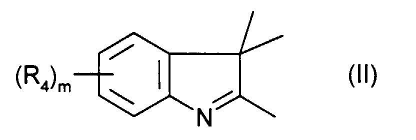 in which R₄ and m are as defined in any one of claims 1 to 3, to react, with heating, with an electrophilic compound of formula (III): 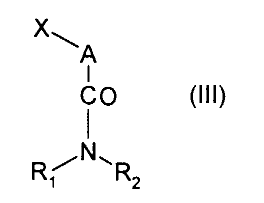 in which A, R₁ and R₂ are as defined in any one of claims 1 to 3, and X is a leaving group, to provide an iminium salt of formula (IV) : 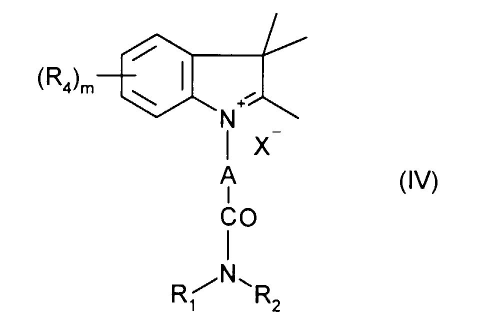
b) allowing said iminium salt of formula (IV) to react with a 1-nitroso-2-naphthol derivative of formula (V) : 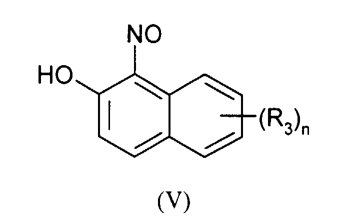 in which R₃ and n are defined in any one of claims 1 to 3, under basic conditions, to provide a compound of formula (I), in which A, R₁, R₂, R₃, R₄, n and m are as defined in any one of claims 1 to 3.

6. A (co)polymer and/or reticulate obtained by polymerisation and/or cross-linking and/or grafting of at least one compound according to any one of claims 1 to 4.

7. A photochromic compound characterised in that it consists of a compound according to any one of claims 1 to 4, or of a mixture of at least two compounds according to any one of claims 1 to 4 or of a mixture of at least one compound according to any one of claims 1 to 4 with at least one other photochromic compound of another type and/or at least one non-photochromic colouring agent.

8. A photochromic composition, characterised in that it comprises :
- at least one compound according to any one of claims 1 to 4 and/or at least one (co)polymer and/or reticulate according to claim 6,
- and, optionally, at least one other photochromic compound of another type and/or at least one non-photochromic colouring agent and/or at least one stabilising agent.

9. A (co)polymer matrix, characterised in that it comprises :
- at least one compound according to any one of claims 1 to 4,
- and/or at least one co(polymer) and/or reticulate according to claim 6,
- and/or at least one composition according to claim 8.

10. The matrix according to 8, characterised in that the (co)polymer is selected from the following list :
- optionally halogenated alkyl, cycloalkyl, aryl or aralkyl poly[mono-, di-, tri-, tetra-]acrylate or poly[mono-, di-, tri-, tetra-]methacrylate or having at least one ether and/or ester and/or carbonate and/or carbamate and/or thiocarbamate and/or urea and/or amide group,
- polystyrene, polyether, polyester, polycarbonate, polycarbamate, polyepoxy, polyurea, polyurethane, polythiourethane, polysiloxane, polyacrylonitrile, polyamide, aliphatic or aromatic polyester, vinylic polymer, cellulose acetate, cellulose triacetate, cellulose acetate-propionate or polyvinylbutyral,
- copolymers of two or more types of monomers or mixtures of polymers mentioned above.

11. An ophthalmic or solar article comprising :
- at least one compound according to any one of claims 1 to 4,
- and/or at least one (co)polymer and/or reticulate according to claim 6,
- and/or at least one composition according to claim 8,
- and/or at least one matrix according to one of claims 9 or 10.

12. The article according to claim 11, characterised in that it is constituted by a lens.

13. A glazing and/or optical device comprising :
- at least one compound according to any one of claims 1 to 4,
- and/or at least one (co)polymer and/or reticulate according to claim 6,
- and/or at least one composition according to claim 8,
- and/or at least one matrix according to one of claims 9 or 10.

14. Intermediate compounds of formula (IV) : 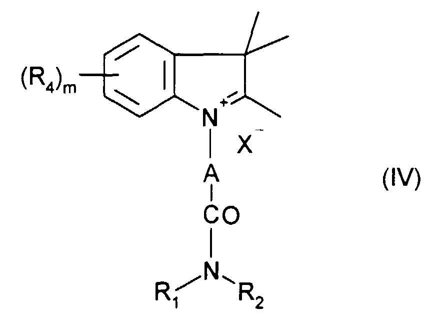 in which R₁ , R₂ , R₄ , m , and A are as defined in claim 1, and X⁻ is a leaving group.
